# EUROPÄISCHE PATENTSCHRIFT

(11) **EP 1 952 804 B1**
(45) Veröffentlichungstag und Bekanntmachung des Hinweises auf die Patenterteilung: **13.03.2013**
(21) Anmeldenummer: 08100465.7
(22) Anmeldetag: 15.01.2008
(51) Int. Cl.: A61K 9/00, A61K 47/12, A61K 47/38, A61K 47/02, A61K 47/36

(54) **Wundheilmittel**
Injury treatment agent
Produit de guérison des plaies

(30) Priorität: 25.01.2007 DE 102007004663
(43) Veröffentlichungstag der Anmeldung: 06.08.2008
(73) Patentinhaber: Bauer, Wulf, 50968 Köln (DE)
(72) Erfinder: Thöne, Gerd, 32105 Bad Salzuflen (DE)
(74) Vertreter: Bauer, Wulf

(56) Entgegenhaltungen:
- EP-A- 0 919 220
- WO-A-97/35558
- DE-U1-202005 007 363
- US-A- 4 536 399
- US-A- 5 785 977
- DATABASE BIOSIS [Online] BIOSCIENCES INFORMATION SERVICE, PHILADELPHIA, PA, US; 1992, OBERBAUM M ET AL: "Wound healing by homeopathic silica dilutions in mice" XP002476360 Database accession no. PREV199395017090 & HAREFUAH, Bd. 123, Nr. 3-4, 1992, Seiten 79-82, 154, ISSN: 0017-7768

## Beschreibung

Die vorliegende Erfindung betrifft ein Wundheilmittel.

Unter einer Wunde wird hier eine Verletzung der Haut verstanden, insbesondere unterschiedlich tiefe Wunden, sowie keimfreie oder mit Keimen behaftete Wunden (aseptische oder septische Wunden).

Das Ziel jeder Wundbehandlung ist es, die Wunde rasch zum Heilen zu bringen. O-berstes Ziel ist es daher, zu verhindern, dass sich eine aseptische Wunde infiziert. Umgekehrt muss eine septische Wunde gesäubert werden, um die Keime zu entfernen.

Die Wundbehandlung wird seit Jahrhunderten erforscht und stetig verbessert. Beispielsweise war das Verbinden und Abdichten von Wunden mit Wundverbänden lange Zeit sehr verbreitet, um eine Infektion mit Wundbakterien durch den Verschluss der Wunde zu vermeiden. Der Verband sollte außerdem das Wundsekret aufsaugen. Dies führt aber häufig zum Austrocknen der Wunde, wodurch sich der normale Wundheilungsprozess sogar verlangsamte.

Moderne Wundverbände dagegen gehen weit über diese einfache Schutzfunktion hinaus, sie beeinflussen die physiologischen Wundheilungsprozesse positiv, indem sie ein Wundmilieu schaffen, dass dem natürlichen Milieu entspricht. Dieses natürliche Wundmilieu ist feucht, damit die Zellteilung und die Zellwanderung positiv beeinflusst und damit die Wundheilung gefördert wird.

Heutzutage wird auf Wunden bevorzugt ein Wundheilmittel in Form eines Sprays oder als Salbe aufgebracht. Dabei kann die Wunde zusätzlich locker, also luftdurchlässig verbunden werden, wobei eine Barriere nach außen nicht notwendig ist bzw., sogar vermieden werden sollte.

Als Wundheilmittel eignen sich zahlreiche unterschiedliche natürliche oder chemische Stoffe. Beispielsweise werden oftmals Pflanzen oder Pflanzeninhaltsstoffe genutzt, die das Abheilen der Wunde beschleunigen.

Wundheilmittel, die auf pflanzlicher Basis basieren, haben insbesondere den Nachteil, dass die entsprechenden Pflanzen in ausreichender Menge stets vorhanden sein müssen. Die Pflanzen müssen also gesät und aufgezogen werden, wobei möglicherweise Jahreszeiten und klimatische Bedingungen die Aufzucht erschweren oder zumindest aufgrund der Einrichtung entsprechender Gewächshäuser hohe Kosten für die Zucht entstehen. Bekannt sind auch pulverförmige Medikamente, die ein Zeolith oder ein Aluminiumsilikat enthalten. Diese werden als Pulver auf die Wunden gestreut. Bei letzteren ist allerdings die Wirksamkeit sehr umstritten und auch das Bestreuen von offenen Wunden mit Pulvern wird in Fachkreisen als problematisch angesehen.

Die Aufgabe der vorliegenden Erfindung besteht darin, ein Wundheilmittel zu schaffen, das in Form eines Sprays oder als Salbe verwendet werden kann. Das Wundheilmittel soll kostengünstig und einfach herzustellen sein und eine gute Wundheilwirkung erzielen. Insbesondere soll das Wundheilmittel möglichst aus Bestandteilen bestehen, die in der Natur selbst vorkommen und in ausreichender Menge und zu jeder Jahreszeit zur Verfügung stehen.

Erfindungsgemäß wird die Aufgabe durch ein Wundheilmittel nach Anspruch 1 gelöst, das als wesentliche Bestandteile Wasser und Kieselsäure mit einer Partikelgröße von 50 bis 200 nm enthält.

Als Kieselsäuren werden die Sauerstoffsäuren des Siliziums bezeichnet, wobei im Sinne dieser Anmeldung auch sämtliche anderen Formen von synthetischen Siliziumdioxiden unter dem Begriff Kieselsäure verstanden werden. Kristallisierte Kieselsäure kommt in der Natur als Cristobalit, Quarz und Tritomit vor. Industriell werden Kieselsäure als Hydrit-Produkte, sogenannte Kieselgele, gefällte Kieselsäuren und pyrogene Kieselsäuren großtechnisch hergestellt. Kieselgele und gefällte Kieselsäuren erhält man durch Reaktion von Wasserglas mit Schwefelsäure in saurem bzw. basischem Milieu. Pyrogene Kieselsäuren werden durch Reaktion von Siliziumtetrachloryd mit Wasser, welches in einer Wasserstoffflamme gebildet wird, hergestellt, wobei Chlorwasserstoffgas entweicht.

Als Wasser eignet sich z.B. normales Trinkwasser, Leitungswasser oder demineralisiertes Wasser oder auch aqua at injectabile.

Dadurch dass die Partikelgröße der Bestandteil lediglich 50 bis 200 nm beträgt, wird die heilsame Wirkung des Wundheilmittels aufgrund der Oberflächenvergrößerung noch verstärkt. Das Wundheilmittel wird auch nicht trocken auf die Wunde gebracht, sondern in Form eines Gels oder Sprays aufgetragen. Die Wunde wird dadurch insbesondere auch vor dem Austrocknen bewahrt.

Der Anteil an Kieselsäure beträgt etwa 1 bis 5 Gew.%, vorzugsweise 2 Gew.% bzw. etwa 5 Vol.% zur Flüssigkeit.

Vorteilhafterweise beträgt der PH-Wert des Wundheilmittels etwa PH 8. Dies hat den Vorteil, dass die Lösung auch über einen längeren Zeitraum stabil bleibt und sich die einzelnen Bestandteile nicht absetzen bzw. trennen. Der PH-Wert wird vorzugsweise durch Zugabe entsprechender Pufferlösungen, wie beispielsweise Calciumhydroxid, Natriumhydroxid oder Zitronensäure eingestellt.

Weiterhin kann ein Konservierungsmittel zugefügt werden, um das Wundheilmittel auch über einen möglichst langen Zeitraum einsatzfähig zu halten. Als Konservierungsmittel hat sich beispielsweise Kaliumsorbat oder Benzoesäure als günstig erwiesen.

Wie bereits ausgeführt, kann die Kieselsäure als reine pyrogene Kieselsäure sowie als gefällte oder kristalline Kieselsäure verwendet werden.

Wesentlich ist, dass die Mischung in einer speziellen Kolloidmühle derart aufgemahlen wird, dass die Bestandteile die gewünschte Größe 50-200 nm, vorzugsweise 120 nm bekommen. Die erfindungsgemäße Lösung kann als Stammlösung verwendet werden, die zu geeigneten Endprodukten weiter verarbeitet werden kann, sie kann aber auch selbst bereits als Wundheilmittel eingesetzt werden.
Zur Schaffung eines erfindungsgemäßen medizinischen Sprays, ist darauf zu achten, dass sich die Bestandteile auch bei längerem Nichtgebrauch nicht derart verändern, dass sie als Sprühmittel nicht mehr eingesetzt werden können. Es besteht beispielsweise die Gefahr, dass sich die Mineralien auf den Grund des Behälters absetzen und sich dann der Sprühkopf einer entsprechenden Sprühflasche schnell zusetzt.
Um dies zu vermeiden, ist eine Stabilisierung durch Zugabe von Schichtsilikaten, wie Magnesiumsilikat und Aluminiumsilikat, Hektorit oder Bentonit aus der Gruppe der Montmorillonite und Methylcellulose (hoch- oder niedrigviskos) möglich.

Die Cellulose verdickt die Lösung bis zu einem Grad, dass die Lösung als solche dauerhaft sprühbar bleibt. Da sie trotzdem noch dazu neigt, sich abzusetzen, wird weiterhin das Schichtsilikat hinzugegeben, welches die Lösung gelförmig macht. Diese bleibt aber trotzdem sprühfähig, weil die gelartige Lösung nur scheinviskos ist, sie also durch Bewegung, z.B. Schütteln ihre Gelform verliert und wieder dünnflüssig wird. Wieder in Ruhe, bildet sich das stabile Gel sofort wieder aus und verhindert das Separieren der Lösung.

Alternativ können auch Xanthan Gum und Johannisbrotkernmehl und Schichtsilikate zur Stammlösung hinzugegeben werden. Auch diese Mischung ist in einem hohen Grad scheinviskos, sie liegt im Ruhezustand als stabiles Gel vor, bei der kleinsten Bewegung wird sie aber dünnflüssig und versprühbar.

Alternativ zu einem Spray kann die Stammlösung auch zu einem Wundgel weiterverarbeitet werden. Dies wird insbesondere durch Hinzugabe von Schichtsilikaten (z.B. Hektorit oder Bentonit) erreicht. Es hat sich gezeigt, dass zu 100 kg Stammlösung etwa 2 bis 6 kg Schichtsilikate ausreichen, um die gewünschte Wirkung zu erzielen. Die Mischung wird in einer speziellen Koloitmühle bei etwa 50° gemahlen, bis sich ein stabiles Gel bildet, das sogleich gebrauchsfertig abgefällt werden kann. Die Viskosität wird dabei vorzugsweise derart eingestellt, dass das Produkt noch pumpbar bleibt. Ein solches Gel lässt sich hervorragend auf Wunden- oder Wundverbände auftragen, weil es fließfähig wird, wenn es aufgestrichen wird, sich aber sofort ein weniger fließfähiges Gel ausbildet, wenn das Wundgel zur Ruhe kommt. Dadurch verbleibt es auf der Wunde und am Wundverband und läuft nicht ab.

Als Stammlösung hat sich insbesondere folgende Zusammensetzung als geeignet erwiesen:
95,00 kg Wasser
2,00 kg Kieselsäure
0,2 kg Kalium Sorbatoder Benzoesäure
0,1 kg Calzium- oder Natriumhydroxyd
0,001 kg Yttriumoxyd.

### Eventuell weitere Additive:

Der Stoff bewirkt eine elektrische Ladung, wodurch eine Abstoßung der einzelnen Partikel erreicht wird, was eine Verklumpung ebenfalls verhindert. Das Wundheilmittel ist jedoch auch ohne Yttriumoxyd voll einsatzfähig.

Es hat sich gezeigt, dass anstelle von Kieselsäure auch Zeolith einsetzbar ist. Hier hat sich insbesondere Klinoptilolith als geeignet erwiesen. Dabei wird nicht die gesamte Kieselsäure durch Klinoptilolith ersetzt, vielmehr bleibt etwa 1 % reinste Kieselsäure in der Mischung, um ein Separieren der Stammlösung zu vermeiden.

Die Erfindung ist nicht auf die beschriebenen Ausführungsbeispiele beschränkt, sondern umfasst alle weiteren Ausführungen, die in den Schutzbereich der nachfolgenden Patentansprüche fallen.

## Patentansprüche

1. Wundheilmittel mit den wesentlichen Bestandteilen Wasser und Kieselsäure mit einer Partikelgröße von 50 bis 200 nm **dadurch gekennzeichnet, dass** es folgende Zusammensetzung aufweist:
etwa 95,00 kg Wasser
etwa 2,00 kg Kieselsäure
etwa 0,2 kg Kalium Sorbat
etwa 0,1 kg Calzium- oder Natriumhydroxyd
0,001 kg Yttriumoxid.

2. Wundheilmittel nach Anspruch 1, **dadurch gekennzeichnet, dass** die Bestandteile eine Partikelgröße von etwa 120 nm aufweisen.

3. Wundheilmittel nach Anspruch 1 oder Anspruch 2, **dadurch gekennzeichnet, dass** der Anteil an Kieselsäure etwa 1 bis 5 Gew.% beträgt.

4. Wundheilmittel nach einem der Ansprüche 1 bis 3, **dadurch gekennzeichnet, dass** der PH-Wert etwa 8 beträgt.

5. Wundheilmittel nach einem der Ansprüche 1 bis 4, **dadurch gekennzeichnet, dass** es weiterhin ein Konservierungsmittel beinhaltet.

6. Wundheilmittel nach einem der Ansprüche 1 bis 6, **dadurch gekennzeichnet, dass** es scheinviskos ist.

7. Wundheilmittel nach einem der Ansprüche 1 bis 7, **dadurch gekennzeichnet, dass** es weiterhin Schichtsilikat beinhaltet.

8. Wundheilmittel nach einem der Ansprüche 1 bis 8, **dadurch gekennzeichnet, dass** es weiterhin Cellulose beinhaltet.

9. Wundheilmittel nach einem der Ansprüche 1 bis 8, **dadurch gekennzeichnet, dass** es weiterhin Xanthan Gum und Johannisbrotkernmehl beinhaltet.

10. Wundheilmittel nach Anspruch 1, **dadurch gekennzeichnet, dass** anstelle von Kalium-Sorbat Benzoesäure verwendet wird.

## Claims

1. A wound-healing agent having the main constituents of water and silicic acid with a particle size of 50 to 200 nm, **characterised in that** said agent has the following composition:
approximately 95.00 kg of water
approximately 2.00 kg of silicic acid
approximately 0.2 kg of potassium sorbate
approximately 0.1 kg of calcium hydroxide or sodium hydroxide
0.001 kg of yttrium oxide.

2. The wound-healing agent according to Claim 1, **characterised in that** the constituents have a particle size of approximately 120 nm.

3. The wound-healing agent according to Claim 1 or Claim 2, **characterised in that** the proportion of silicic acid is approximately 1 to 5 % by weight.

4. The wound-healing agent according to one of Claims 1 to 3, **characterised in that** the pH value is approximately 8.

5. The wound-healing agent according to one of Claims 1 to 4, **characterised in that** it further contains a preservative.

6. The wound-healing agent according to one of Claims 1 to 5, **characterised in that** it has apparent viscosity.

7. The wound-healing agent according to one of Claims 1 to 6, **characterised in that** it further contains phyllosilicate.

8. The wound-healing agent according to one of Claims 1 to 7, **characterised in that** it further contains cellulose.

9. The wound-healing agent according to one of Claims 1 to 8, **characterised in that** it further contains xanthan gum and locust bean gum.

10. The wound-healing agent according to Claim 1, **characterised in that** benzoic acid is used instead of potassium sorbate.

## Revendications

1. Agent de cicatrisation dont les composants essentiels sont l'eau et la silice ayant une taille des particules comprise entre 50 et 200 nm, **caractérisé en ce qu'**il présente la composition suivante :
environ 95,00 kg d'eau
environ 2,00 kg de silice
environ 0,2 kg de sorbate de potassium
environ 0,1 kg d'hydroxyde de calcium ou de sodium
0,001 kg d'oxyde d'yttrium.

2. Agent de cicatrisation selon la revendication 1, **caractérisé en ce que** les composants présentent une taille des particules d'environ 120 nm.

3. Agent de cicatrisation selon la revendication 1 ou la revendication 2, **caractérisé en ce que** la proportion de silice est d'environ 1 à 5 en poids.

4. Agent de cicatrisation selon l'une des revendications 1 à 3, **caractérisé en ce que** le pH est d'environ 8.

5. Agent de cicatrisation selon l'une des revendications 1 à 4, **caractérisé en ce qu'**il contient en outre un agent conservateur.

6. Agent de cicatrisation selon l'une des revendications 1 à 5, **caractérisé en ce qu'**il présente une viscosité apparente.

7. Agent de cicatrisation selon l'une des revendications 1 à 6, **caractérisé en ce qu'**il contient en outre du phyllosilicate.

8. Agent de cicatrisation selon l'une des revendications 1 à 7, **caractérisé en ce qu'**il contient en outre de la cellulose.

9. Agent de cicatrisation selon l'une des revendications 1 à 8, **caractérisé en ce qu'**il contient en outre de la gomme xanthane et de la farine de graines de caroube.

10. Agent de cicatrisation selon la revendication 1, **caractérisé en ce que** l'en utilise de l'acide benzoïque au lieu du sorbate de potassium.
